# EUROPÄISCHE PATENTSCHRIFT

(11) **EP 1 808 696 B1**
(45) Veröffentlichungstag und Bekanntmachung des Hinweises auf die Patenterteilung: **11.03.2009**
(21) Anmeldenummer: 07000476.7
(22) Anmeldetag: 11.01.2007
(51) Int. Cl.: G01N 33/543

(54) **Immunologisches Testelement mit verbesserter Kontrollzone**
Immunological test element with improved control zone
Elément de test immunologique doté d'une zone de contrôle améliorée

(30) Priorität: 14.01.2006 EP 06000791
(43) Veröffentlichungstag der Anmeldung: 18.07.2007
(73) Patentinhaber: Roche Diagnostics GmbH, 68305 Mannheim (DE); F.HOFFMANN-LA ROCHE AG, 4070 Basel (CH)
(72) Erfinder: Klepp, Juergen, 76676 Graben-Neudorf (DE)

(56) Entgegenhaltungen:
- WO-A-00/31538
- WO-A-00/77524
- US-A1- 5 939 272
- US-A1- 2002 055 126
- US-A1- 2005 118 662

## Beschreibung

Die Erfindung betrifft ein Testelement zur Durchführung eines immunologischen Sandwichtestes zur Bestimmung eines Analyten aus einer flüssigen Probe mit einer neuartigen Kontrollzone, vorzugsweise in Form von immunochromatographischen Testvorrichtungen (Teststreifen) oder immunologischen (mikrofluidischen oder Kapillarspalt-)Testelementen.

Diagnostische Assays zur Untersuchung von flüssigen Proben wie Blut, Serum, Plasma, Urin, Speichel, Schweiß etc. enthalten oftmals einen Kontrollmechanismus, der die ordnungsgemäße Durchführung des Testes und dessen Funktionsfähigkeit sicherstellen soll.

Bei der Durchführung diagnostischer Tests auf Laborautomaten ist ein testinterner Kontroll-mechanismus eher unüblich, da die ordnungsgemäße Testdurchführung üblicherweise vom Gerät selbst gewährleistet und/oder überwacht wird. Bei Laborautomaten werden beispielsweise die Pipettiervolumina oder die Inkubationszeiten und andere Größen meist automatisch vom Analysenautomaten überwacht. Die Funktionsfähigkeit des Testes (sowohl hinsichtlich der Reagenzien als auch hinsichtlich der Geräte) wird üblicherweise durch die Mitbestimmung von Kontrollproben innerhalb einer Messserie sichergestellt.

Im Point-of-Care-(PoC)-Umfeld, d. h. bei Messungen von Patientenproben außerhalb eines spezialisierten Labors, werden oftmals Einzelbestimmungen durchgeführt. Die dabei verwendeten Testelemente enthalten meist getrocknete Reagenzien, die durch die Probe wieder aufgelöst werden und so für die eigentliche Analyse reaktiviert werden. Insbesondere im Bereich der Immunoassays haben sich im PoC-Umfeld immunologische Testelemente, wie z. B. immunchromatographische Teststreifen oder immunologische Kapillarspalttestelemente, als besonders brauchbar erwiesen. Die immunologischen Testelemente beruhen dabei oftmals auf optisch nachweisbaren Reaktionen, die meist visuell oder mittels Messgerät (Reader) ausgewertet werden. Dem Testdurchführenden kommt in diesem Fall eine besondere Verantwortung zu, da Funktionen, die bei nasschemischen Testen vom Laborautomaten ausgeführt werden, zum Teil vom Testdurchführenden übernommen werden müssen (beispielsweise das Pipettieren des korrekten Probevolumens oder die Einhaltung der Auswertezeit).

Üblicherweise handelt es sich beim durchführenden Personal im PoC-Umfeld meist jedoch nicht um speziell geschulte Mitarbeiter. Tests im PoC-Bereich werden häufig von Ärzten, Krankenschwestern, Arzthelferinnen, Apothekern oder dem Patienten selbst durchgeführt. In spezialisierten Labors (z. B. den Zentrallabors in Krankenhäusern, Großlabors) hingegen werden die Assays von speziell ausgebildeten technischen Assistenten (z. B. MTA) durchgeführt.

Testanbieter haben daher für diese speziellen "dezentralen" oder PoC-Anwendungen so genannte "on-board-controls" entwickelt, die bei der Durchführung auf grobe Handhabungsfehler (beispielsweise Unterdosierung) und/oder fehlende Funktionsfähigkeit des Tests ansprechen und den Anwender damit auf ein ungültiges Testergebnis aufmerksam machen.

Im speziellen Fall der immunologischen Testelemente, die nach dem so genannten SandwichPrinzip arbeiten, sind "on-board-controls" üblicherweise in Form von Kontrollstrichen oderzonen (beide Begriffe sollen nachfolgend synonym verwendet werden) realisiert.

Diese Kontrollstriche oder -zonen dienen im Idealfall dazu, sicherzustellen, dass der Anwender erkennt, dass genügend Probevolumen zur korrekten Testdurchführung aufgetragen wurde, dass die im Testelement imprägnierten oder getrockneten immunchemischen Reagenzien noch in migrationsfähiger (d. h. im Testelement beweglicher) Form vorgelegen haben und dass die immunologische Aktivität der funktionskritischen Reagenzien noch - innerhalb eines gewissen Rahmens - gegeben war. Andernfalls erlaubt der Kontrollstrich bzw. die Kontrollzone ein Erkennen der unsachgemäßen Testdurchführung oder der Unbrauchbarkeit des Testelements und hilft so zu verhindern, dass falsche Testergebnisse erhalten werden.

Ein typischer Testaufbau ist dabei wie folgt:
Zwischen einer Probenauftragszone und einer Nachweiszone befindet sich üblicherweise eine Konjugatzone (auch: Reagenzzone) in oder auf dem Testelement. Probenauftragszone und Konjugatzone können auch identisch sein. Die Konjugatzone enthält einen durch die Probenflüssigkeit wieder auf- und ablösbaren und so mobilisierbaren, gegen den Analyt gerichteten immunologischen Bindepartner (typischerweise einen Antikörper oder ein Fragment hiervon), der üblicherweise an ein signalgenerierendes Enzym oder ein so genanntes Direktlabel (z. B. Gold- oder farbige Latexpartikel, Fluoreszenzlabel) konjugiert ist (so genanntes Konjugat).
Die Nachweiszone enthält einen irreversibel immobilisierten zweiten unabhängigen, gegen den Analyt (oder einen vom Analyten gebildeten Komplex) gerichteten Bindepartner.
In Anwesenheit von Analyt in der Probe wird dieser daher in Form eines sogenannten Sandwiches (d. h. eines immunologischen Komplexes aus immobilisiertem Bindepartner, Analyt und signalgebendem Bindepartner) in der Nachweiszone angereichert und - gegebenenfalls nach Zugabe weiterer Reagenzien - sichtbar.
Beim Streptavidin-Biotin-Prinzip (als Stellvertreter für das so genannte indirekte Sandwich-Prinzip) befindet sich der zweite Bindepartner in Form eines mobilisierbaren Biotin-Konjugates mit in der Konjugatzone. Die Nachweiszone enthält irreversibel immobilisiertes Streptavidin.
Überschüssiges Konjugat aus Bindepartner und Label oder Biotin wird zusammen mit der Probe in einen "flussabwärts" zur Nachweiszone liegenden Waste (Saugzone, die zur Aufnahme überschüssiger Probe dient) transportiert.
Zwischen Nachweiszone und Waste befindet sich üblicherweise die Kontrollzone.

Die folgenden Funktionsprinzipien an Kontrollzonen sind dem Fachmann bekannt und werden mit ihren Vor- und Nachteilen beschrieben:
1. Analyt-Kontrollzone
   Die zwischen der Nachweiszone und dem Waste gelegene Kontrollzone enthält irreversibel immobilisierten Analyt (oder ein Analytanalogon). Überschüssiges Bindepartner-Label-Konjugat wird am immobiliserten Analyten bzw. Analytanalogon gebunden und führt so zu einem nachweisbaren Signal am Kontrollstrich.
   Dieser Mechanismus wird heute oftmals durch Polyhapten-Kontrollzonen abgedeckt, daher sind Vor- und Nachteile unter Punkt 2 näher beschrieben.
   Ein spezieller Nachteil des Analyt-Kontrollstriches ist, dass der Analyt, z. B. ein Antigen, oft schwer und nur aufwendig in ausreichender Menge herstellbar ist.
2. Polyhapten-Kontrollzone
   Die zwischen der Nachweiszone und dem Waste gelegene Kontrollzone enthält ein gegen den Konjugatantikörper gerichtetes Polyhapten.
   Vorteil: Neben der Migrationsfahigkeit der Reagenzien und der Kontrolle des korrekten Probevolumens gewährleistet die Ausbildung des Signals in der Kontrollzone auch die immunologische Aktivität des Konjugates aus Analyt-Bindepartner und Label. Nachteil: Bei hoher Analytkonzentration in der Probe ist die Antigenbindefähigkeit des Bindepartner-Label-Konjugates vor Erreichen der Kontrollzone bereits gesättigt und das Polyhapten in der Kontrollzone kann kein Konjugat mehr abfangen. Der Kontroll-mechanismus versagt folglich.
   In diesem Fall muss der Anwender fälschlicherweise von einem invaliden Testergebnis in der Nachweiszone ausgehen.
   Dies ist besonders häufig dann ein Nachteil, wenn zwischen cut-off-Wert des Testes (d. h. der unteren Messbereichsgrenze) und dem oberen Konzentrationsbereich eine weite Konzentrationsspanne abgedeckt werden muss. Dies ist z. B. bei Schwangerschaftstests der Fall, wo aus Urin das Hormon hCG mit einem cut-off von ca. 10 - 20 mIU/ml nachgewiesen werden muss, andererseits auch hCG-Werte bis 500.000 mIU/ml am Ende des ersten Schwangerschaftstrimesters beobachtet werden. Solch hohe Analytkonzentrationen führen zu negativen Ergebnissen in der Kontrollzone und täuschen so ein invalides Testergebnis in der Nachweiszone vor.
3. Anti-IgG-Kontrollzone (als eine Form der indirekten Kontrollzone)
   Die Kontrollzone enthält einen gegen das Antikörper-Label-Konjugat gerichteten Antikörper. Dieses Kontrollzonenprinzip ist sehr verbreitet bei Schwangerschaftsteststreifen.
   Beispiel: Bei Verwendung eines analytspezifischen, gelabelten Mausantikörpers enthält die Kontrollzone z. B. einen gegen Mausantikörper gerichteten polyklonalen Antikörper (z. B. PAK<MausFcγ>S-IgG).
   Vorteil: Die Ausbildung des Kontrollstriches wird durch hohe Analytkonzentrationen der Probe nicht beeinflusst.
   Nachteil: Die korrekte immunologische Aktivität des Konjugates wird durch diesen Mechanismus nicht erfasst. Dies führt in jüngster Zeit bei Neuzulassungen von Testelementen des Öfteren zu Diskussionen mit den Zulassungsbehörden, z. B. der FDA in den USA. Ein weiterer Nachteil liegt darin, dass bei Verwendung von Entstörantikörpern in den Assays, die mit Blut arbeiten, die Intensität des Signals in der Kontrollzone stark herabgesetzt wird. Entstörantikörper werden im Test, bezogen auf den analytspezifischen Konjugatantikörper, üblicherweise in einem 10- bis 500-fachen Überschuss eingesetzt. Als Entstörantikörper werden dabei üblicherweise unkonjugierte Antikörper der gleichen Spezies eingesetzt, die somit vom Kontrollstrich ebenfalls abgefangen werden.
   Bei Anwesenheit von Analyt in der Probe kommt erschwerend hinzu, dass bereits ein Großteil des Konjugates in der Nachweiszone bindet und die oft geringe Restkonjugatmenge in Anwesenheit des Entstörantikörpers zum kompletten Versagen der Kontrollzone führt.
4. Indirekter, von Entstörantikörpern unbeeinflusster Kontrollstrich
   Der Kontrollstrich enthält einen Bindepartner, der gegen einen "unabhängigen Label" des Konjugates gerichtet ist.
   Beispiel: Der analytspezifische Bindepartner des Konjugates wird zusätzlich digoxigeniliert und über einen anti-Digoxigenin-Antikörper am Kontrollstrich abgefangen (vgl. hierzu beispielsweise US-A 2002-0055126).
   Vorteil: Die Bindefähigkeit des Kontrollstriches wird nicht durch hohe Analytkonzentrationen herabgesetzt. Des Weiteren wird die Bindefähigkeit des Kontrollstriches nicht durch die Anwesenheit von Entstörantikörpern herabgesetzt.
   Nachteil: Die Antikörper des Konjugates müssen zusätzlich speziell modifiziert werden, was zusätzlichen Aufwand, Kosten und Zeit bedeutet. Des Weiteren kann bei hohem endogenen Gehalt des gewählten Labels der Kontrollstrich wiederum versagen.

Aus dem beschriebenen Stand der Technik ist ersichtlich, dass Bedarf nach einem verbesserten Kontrollstrich-Mechanismus existiert.

Die Aufgabe der vorliegenden Erfindung ist es, die Nachteile des Standes der Technik zu beseitigen. Insbesondere ist es die Aufgabe der Erfindung, für immunologische Testelemente einen Kontroll-Mechanismus bereitzustellen, der unabhängig von Rezepturkomponenten des Testes (insbesondere hinsichtlich der Verwendung von so genannten Entstörantikörper) über einen weiten Konzentrationsbereich des Analyten zuverlässig funktioniert, ohne von endogenen Probebestandteilen beeinflusst zu werden.

Diese Aufgabe wird durch den Gegenstand der Erfindung gelöst.

Gegenstand der Erfindung ist ein Testelement gemäß Anspruch 1 sowie ein Verfahren gemäß Anspruch 10. Bevorzugte Ausführungsformen der Erfindung sind Gegenstand der abhängigen Ansprüche.

Gegenstand der Erfindung ist zunächst ein Testelement zur Durchführung eines immunologischen Sandwichtestes zur Bestimmung eines Analyten aus einer flüssigen Probe, wie es - mit Ausnahme der neuartigen Kontrollzone - prinzipiell aus dem Stand der Technik in vielen Varianten bekannt ist. Beispielsweise kann das Testelement im Wesentlichen aus saugfähigen Materialien wie Vliesen, Membranen, Papieren etc. aufgebaut sein (vergleiche beispielsweise US 4,861,711, US-A 2002-0055126, WO 2005/111607) oder den Flüssigkeitstransport über mikrofluidische Strukturen (teilweise auch durch externe Krafteinwirkung wie Saugen, Drücken, Zentrifugieren getrieben) oder Kapillarkanäle bewerkstelligen (vergleiche beispielsweise US 6,156,270)

Das Testelement enthält eine Reagenzzone, die ein durch die flüssige Probe lösliches Konjugat aus einem Analyt-Bindepartner (typischerweise - falls der Analyt ein Antigen oder Hapten isteinem Antikörper oder einem immunologisch aktiven, zur Analytbindung befähigten Antikörperfragment, bzw. - falls der Analyt ein Antikörper ist - einem Antigen oder Hapten) und einem Label, das direkt oder indirekt visuell, optisch oder elektrochemisch nachweisbar ist, enthält. Geeignete Label sind beispielsweise Enzyme, Fluoreszenzlabel, elektrochemisch aktive Gruppen oder so genannte Direktlabel wie Metall- oder Kohlenstofflabel oder gefärbte Latices. Diese Zone wird auch als Konjugatzone bezeichnet.

Die Konjugatzone kann als Probenaufgabezone dienen oder einer separaten Probenaufgabezone vor- oder nachgelagert sein. Sie kann neben dem oben beschriebenen Konjugat aus Analyt-Bindepartner und Label auch ein weiteres Konjugat aus einem zweiten Analytbindepartner (wiederum typischerweise einem Antikörper oder einem immunologisch aktiven, zur Analytbindung befähigten Antikörperfragment) und einer Markierungssubstanz, die selbst Partner in einem Bindepaar ist, enthalten. Die Markierungssubstanz kann beispielsweise Biotin oder Digoxigenin sein und kann dazu dienen, einen Sandwichkomplex aus gelabeltem Konjugat, Analyt und markiertem Konjugat in der Nachweis- und/oder Kontrollzone zu immobilisieren.

Das Testelement umfasst weiterhin eine Nachweiszone, die einen permanent immobilisierten, d. h. nicht durch die flüssige Probe ablösbaren, Bindepartner für den Analyten oder für den Analyten enthaltende Komplexe enthält. Der immobilisierte Bindepartner ist dabei wiederum typischerweise ein Antikörper oder ein immunologisch aktives, zur Analytbindung befähigtes Antikörperfragment bzw. ein Antigen oder (Poly-)Hapten. Für den Fall, dass eines der oben genannten markierten Konjugate verwendet wird, die z. B. Biotin bzw. Digoxigenin in Verbindung mit einem Analyt-Bindepartner tragen, kann der immobilisierte Bindepartner auch Streptavidin oder Polystreptavidin bzw. ein anti-Digoxigenin-Antikörper sein.

Schließlich befindet sich in oder auf dem Testelement eine Kontrollzone, die einen permanent immobilisierten Bindepartner für das Konjugat aus Analyt-Bindepartner und Label enthält, beispielsweise in Form eines immobilisierten Polyhaptens, das als Analytanalogon fungiert und in der Lage ist, den Analyt-Bindepartner aus dem gelabelten Konjugat zu binden. Erfindungswesentlich ist dabei, dass die Kontrollzone weiterhin einen oder mehrere permanent immobilisierte(n) Bindepartner für den Analyten oder für den Analyten enthaltende Komplexe enthält. Die letztgenannten Bindepartner können dabei aus den gleichen Verbindungen ausgewählt werden, die oben im Zusammenhang mit den immobilisierten Bindepartnern der Nachweiszone beschrieben sind. Typischerweise sind diese immobilisierten Bindepartner in der Nachweiszone und in der Kontrollzone identisch. Es ist jedoch auch möglich, dass sie unterschiedlich sind, beispielsweise dass in der Nachweiszone ein Bindepartner für ein Biotin-markiertes Konjugat immobilisiert ist (also z. B. Polystreptavidin) und in der Kontrollzone - neben dem Polyhapten - ein anti-Analyt-Antikörper. Im letztgenannten Fall sollte der zusätzlich in der Kontrollzone immobilisierte anti-Analyt-Antikörper gegen ein (weiteres) unabhängiges und somit nicht von den Konjugatantikörpern (Biotin-markiertes Konjugat und gelabeltes Konjugat) erkanntes Epitop gerichtet sein.

Ein weiterer Gegenstand der Erfindung ist ein Verfahren zum Nachweis eines Analyten aus einer flüssigen Probe mit Hilfe des erfindungsgemäßen Testelements. Die Probe (die selbst flüssig sein kann oder - falls sie selbst nicht flüssig ist - in einer Flüssigkeit, z. B. Puffer, aufgelöst oder suspendiert werden und so eingesetzt werden kann) wird auf das Testelement aufgegeben und löst beim Erreichen der Reagenzzone (Konjugatzone) die dort in migrationsfähiger Form vorhandenen Reagenzien. In der Konjugatzone wird der Analyt - sofern in der Probe vorhanden - mit dem gelabelten Konjugat in Kontakt gebracht und reagiert zu einem Analyt-Konjugat-Komplex. Die Mischung aus Probe und Reagenz erreicht anschließend die Nachweiszone, wo zumindest ein Teil der Analyt-Konjugat-Komplexe an den permanent immobilisierten Bindepartner für den Analyten oder für den Analyten enthaltende Komplexe bindet. Gelabeltes Konjugat, das nicht mit dem Analyten reagiert hat, bindet in der Kontrollzone an den permanent immobilisierten Bindepartner für das Konjugat. Außerdem bindet der Teil der Analyt-Konjugat-Komplexe, der nicht in der Nachweiszone abgefangen wurde, in der Kontrollzone an den permanent immobilisierten Bindepartner für den Analyten oder für den Analyten enthaltende Komplexe.

Die Erfindung wird anhand des nachfolgenden Beispiels und der Figur näher erläutert.

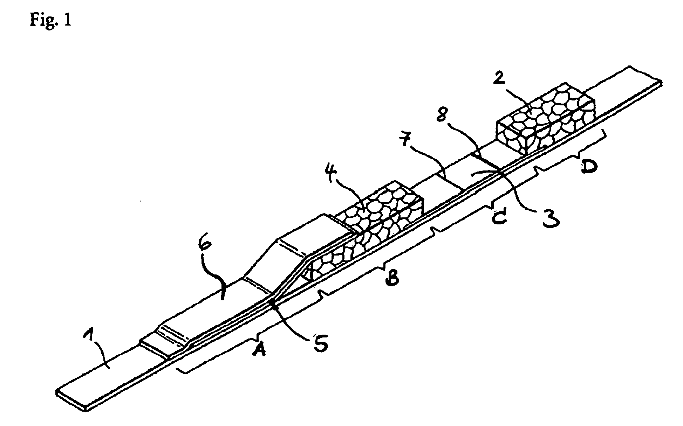

In Figur 1 ist schematisch eine bevorzugte Ausführungsform eines erfindungsgemäßen Testelementes in Form eines immunochromatographischen Teststreifens abgebildet.

Die Ziffern und Buchstaben in der Figur bedeuten:
A Probenaufgabe- und Konjugatzone
B Erythrozytenabtrennzone
C Detektionszone
D Saugzone
1 Trägermaterial
2 Saugvlies (Waste)
3 Chromatographiemembran mit Nachweis- und Kontrollstrich
4 Erythrozytenabtrennmatrix (Blutabtrennvlies)
5 Goldkonjugatvlies (gelabeltes Konjugat)
6 Biotinkonjugatvlies (markiertes Konjugat)
7 Nachweisstrich (immobilisiertes Polystreptavidin)
8 Kontrollstrich

Der in Figur 1 gezeigte Teststreifen entspricht - mit Ausnahme des erfindungsgemäßen Kontrollstrichs - vom Aufbau und der Funktion her den Roche Cardiac T Quantitative (Roche Diagnostics GmbH, Mannheim, Deutschland) Troponin T-Teststreifen. Analoge Teststreifen zur Bestimmung weiterer Analyten sind ebenfalls erhältlich (vgl. beispielsweise US-2005-0118662-A1, wo ein entsprechender NT-proBNP-Teststreifen beschrieben ist).

Auf einem Trägermaterial 1 aus Kunststoff befinden sich nacheinander i) eine Probenaufgabezone A, die ein Vliesmaterial 5 mit darin ablösbar imprägniertem anti-Analyt-Antikörper-Gold-Konjugat und ein Vliesmaterial 6 mit darin ablösbar imprägniertem anti-Analyt-Antikörper-Biotin-Konjugat aufweist, ii) eine Erythrozytenabtrennzone B, die ein Glasfaservlies als Erythrozytenabtrennmatrix 4 enthält, die in der Lage ist, aus Vollblut Erythrozyten abzutrennen und weitgehend farbloses Blutplasma zu erzeugen, iii) eine Detektionszone C, die eine Chromatographiemembran 3 aus Nitrocellulose enthält, in bzw. auf der ein Polystreptavidin-Nachweisstrich 7 und ein Kontrollstrich 8 immobilisiert sind, sowie iv) ein Saugzone D mit einem Saugvlies, das die Wanderung der Probe durch die Chromatographiemembran 3 erleichtert und gleichzeitig zur Aufnahme der gewanderten Probe als Waste dient.

Das anti-Analyt-Antikörper-Gold-Konjugat (auch: gelabeltes Konjugat) in Vlies 5 und das anti-Analyt-Antikörper-Biotin-Konjugat (auch: markiertes Konjugat) in Vlies 6 dienen zur Sandwichausbildung mit dem Analyten; immobilisiertes Poly-Streptavidin in der Membran 3 dient zur Bildung des Nachweisstrichs 7.

Der erfindungsgemäße Kontrollstrich 8 enthält in diesem Fall neben dem permanent immobiliserten Bindepartner für das anti-Analyt-Antikörper-Gold-Konjugat (d. h. einem antikörperspezifischen Polyhapten (oder Analyt)) zusätzlich einen permanent immobilisierten Bindepartner für den Analyten enthaltende Komplexe (d. h. in diesem Fall Polystreptavidin).

Das hat zur Folge, dass bei geringer bis mittlerer Analytkonzentration der Kontrollstrich 8 das gelabelte Konjugat direkt und immunspezifisch hauptsächlich via Polyhapten/Analyt-Konjugat-Bindung abfängt. In diesem Fall funktioniert der Streptavidin-Biotin-Sandwich-Teil des Kontrollstriches 8 noch nicht, da die geringe Menge an sich gebildetem Sandwich nahezu quantitativ "flussaufwärts" bereits vom Nachweisstrich 7 abgefangen wird.

Bei hohen Analytkonzentrationen versagt der Polyhapten-Konjugat-Bindemechanismus des Kontrollstriches 8, da das Konjugat bereits mit dem Analyt der probe gesättigt ist. In diesem Fall wird jedoch in Form des Sandwichkomplexes (aus gelabeltem Konjugat, Analyt und markiertem Konjugat) vorliegendes Konjugat vom Streptavidin-Biotin-Mechanismus des Kontrollstriches 8 abgefangen. Dies funktioniert deshalb, da kinetisch bedingt der Nachweisstrich 7 nicht sämtlichen Sandwichkomplex abfängt und die "flussabwärts" chromatographierenden Anteile vom Kontrollstrich 8 gebunden werden.

Bei extrem hoher Analytkonzentration kann es im Sandwich-Format zum so genannten High-Dose-Hook-Effekt am Nachweisstrich 7 kommen. Die Ursache hierfür lässt sich wie folgt erklären: Bei limitierter Analytmenge (Antigenunterschuss bezüglich der vorhandenen Antikörpermenge im Testelement) bindet ein Antigenmolekül beide Bindepartner (gelabelter und markierter Antikörper) gleichzeitig über unabhängige Epitope. Es kommt so zur Ausbildung des gewünschten Sandwichkomplexes und der Analyt ist sowohl an einen Bindepartner gebunden, der für die Immobilisierung in der Nachweiszone 7 sorgt (hier: biotinylierter Antikörper, der an das Streptavidin binden kann), als auch an einen gelabelten Bindepartner (hier: Gold-markierter Antikörper), der für die Detektierbarkeit des Analyten sorgt. Bei hohem Analytüberschuss (bezogen auf die Menge an gelabeltem und markiertem Konjugat) binden die beiden Sandwichpartner (d. h. gelabeltes Konjugat einerseits und markiertes Konjugat andererseits) jeweils an zwei unabhängig vorliegende Analytmoleküle, so dass in der Nachweiszone 7 zwar Analyt über das markierte Konjugat gebunden, dieser aber aufgrund der fehlende Bindung an ein gelabeltes Konjugat nicht nachgewiesen werden kann.

Tritt ein High-Dose-Hook-Effekt am Nachweisstrich 7 auf, so täuscht dieser trotz hoher Analytkonzentration ein Analyt-negatives Testergebnis vor. Ein analytunabhängiger Kontrollstrich 8 (beispielsweise auf Basis eines immobilisierten PAK<MausFcγ>S-IgG-Antikörpers) zeigt in diesem Fall ein korrektes Funktionieren des Testes an, was letztendlich zu einer falsch negativen Testinterpretation führt. In diesem Fall wäre es eigentlich wünschenswert, dass der Kontrollstrich 8 dem gleichen "High-Dose-Hook-Effekt" unterliegt wie der Nachweisstrich 7 und durch gleichzeitiges Versagen das Testergebnis als invalide (und damit zumindest nicht falsch negativ) angezeigt wird.

Genau dies ist beim vorliegenden neuartigen Kontrollstrich 8 der Fall und ist als weiterer Vorteil anzusehen: Auf Grund der hohen Analytkonzentration der Probe liegt beim Erreichen der Kontrollzone praktisch kein freies, das heißt nicht an Analyt gebundenes, gelabeltes Konjugat mehr vor, so dass der Teil des Kontrollstriches 8, der auf dem permanent immobilisierten Bindepartner für das gelabelte Konjugat (hier: Polyhapten) beruht, in der Probe-Reagenz-Mischung keinen Bindepartner mehr findet. Es wirkt folglich nur noch der Teil des Kontrollstrichs 8, der auf dem permanent immobilisierten Bindepartner für den Analyten bzw. den Analyten enthaltende Komplexe basiert (hier: dem Streptavidin-Biotin-Mechanismus). Wird jetzt die Sandwichausbildung durch besagten High-Dose-Hook-Effekt verhindert, versagt neben dem Nachweisstrich 7 auch der Kontrollstrich 8. Das Testergebnis ist vom Benutzer dann als ungültig und nicht fälschlich als "negativ" zu erkennen.

Die Erfindung kann nicht nur auf das oben im Detail beschriebene "indirekte Sandwichprinzip" unter Verwendung der Streptavidin-Biotin-Bindung angewandt werden. Eine Verallgemeinerung auf das "direkte Sandwichprinzip" ist möglich. In diesem Fall ist ein Sandwichpartner irreversibel in der Nachweisstrichzone immobilisiert. Der Kontrollstrich enthält daher neben dem (gegen den Konjugatantikörper gerichteten) Polyhapten zusätzlich den im Nachweisstrich immobilisierten Sandwich-Antikörper. Es sei darauf hingewiesen, dass in diesem Fall die alternative Verwendung von Antigen anstelle von Polyhapten als immobilisiertem Bindepartner in der Kontrollzone nicht bevorzugt ist, da der in der Kontrollstrichzone gleichzeitig mit dem Antigen aufgebrachte Sandwich-Antikörper bereits einen Antigen-Antikörper-Komplex ausbildet und sich somit beide Teilmechanismen des neuartigen Kontrollstriches zumindest teilweise "neutralisieren" würden. Bevorzugt ist deshalb die Verwendung eines Polyhaptens, da dieses "synthetische Epitop" lediglich gegen den migrationsfähigen Konjugatantikörper, nicht jedoch gegen den zweiten Sandwichantikörper gerichtet ist. Die Funktionsfähigkeit ist somit gegeben.

### Beispiel 1

Die Erfindung wird nachfolgend beispielhaft an einem Teststreifen zum Nachweis von kardialem Troponin I (cTn I oder kurz: Tn I) aus Vollblut beschrieben.

### a.) Analysenelement

Es werden Teststreifen gemäß Figur 1 hergestellt.

Auf einer 5 mm breiten und 78 mm langen Trägerfolie 1 aus Polyester (Melinex, 350 µm dick, von Imperial Chemistry Industries, GB) werden mit Schmelzkleber (Dynapol S 1358 von Hüls AG, Deutschland)
- ein 1,5 mm dickes und 7 mm langes Vlies aus 100 Teilen Glasfaser (Durchmesser 0,49 bis 0,58 µm, Länge 1000 µm) und 5 Teilen Polyvinylalkoholfasern (Kuralon VPB 105-2 von Kuraray) mit einem Flächengewicht von ca. 180 g/m² als Flüssigkeitssammelzone (Waste) 2 (Saugzone D)
- eine 1,5 cm lange Cellulosenitrat-Membran (Typ CN 11301 von Sartorius, Deutschland) als Chromatographiemembran 3 enthaltend Nachweis- und Kontrollzone (Detektionszone C)
- ein 1,5 mm dickes und 13 mm langes Vlies aus 100 Teilen Glasfaser und 10 Teilen Polyvinylalkoholfasern (beide Fasern analog der Flüssigkeitssammelzone) mit einem Flächengewicht von ca. 180 g/m² als Blutabtrennvlies 4 (Erythrozytenabtrennzone B)
- und zwei 18 mm bzw. 20 mm lange Vliese aus 80 Teilen Polyesterfasern, 20 Teilen Zellwolle und 20 Teilen Polyvinylalkoholfasern mit einer Dicke von ca. 0,32 mm und einem Flächengewicht von ca. 80 g/m² (dessen Herstellung in der europäischen Patentschrift EP 0 326 135 in Beispiel 1 beschrieben ist), enthaltend Goldkonjugat (Goldkonjugatvlies 5) als Zone mit markiertem Partner des spezifischen Bindungspaares und enthaltend Biotinkonjugat (Biotinkonjugatvlies 6) als weitere Zone mit markiertem Partner des spezifischen Bindungspaares,
jeweils leicht überlappend nebeneinander befestigt.

Auf die zuvor beschriebene Cellulosenitrat-Membran der Detektionszone 3 wird durch Strichdosierung eine wässrige Streptavidinlösung (4,5 mg/ml) aufgebracht. Hierzu wird die Dosierung so gewählt (Dosiermenge 0,1 ml/min, Bahngeschwindigkeit 3 m/min), dass ein Strich mit einer Breite von ca. 0,4 mm entsteht. Dieser Strich 7 dient zum Nachweis des zu bestimmenden Analyts und enthält ca. 0,8 µg Streptavidin pro Teststreifen.

In einem Abstand von etwa 4 mm flussabwärts vom Streptavidinstrich wird unter identischen Dosierungsbedingungen eine wässrige Tn 1-Polyhapten-Lösung mit 1 mg/ml (Herstellung des Polyhaptens siehe unten), eine Streptavidinlösung mit 1 mg/ml oder eine 1:1 Mischung aus Polyhaptenlösung und Streptavidinlösung (jeweils 1 mg/ml) aufgebracht. Diese Striche 8 dienen als Funktionskontrolle des Teststreifens und enthalten entweder ca. 0,15 µg Polyhapten und/oder 0,15 µg Streptavidin pro Teststreifen.

Die Membran wird anschließend luftgetrocknet.

Das Polyhapten wird wie folgt hergestellt: Ein Peptid mit einer Aminosäuresequenz, die dem Epitop mit den Aminosäuren (aa) 27-43 des kardialen Troponin I entspricht (siehe FEBS, Vol. 270; No. 1, 2; page 57 - 61: "Molecular cloning of human cardiac troponin I using polymerase chain reaction") wird über eine dem Fachmann bekannte klassische Peptide-Festphasensynthese hergestellt. Als N-terminaler Spacer zur Anbindung an das Trägerprotein wird ein Peptidrest bestehend aus β-Alanin, ε-Aminocapronsäure, β-Alanin und Cystein verwendet, so dass das Peptid ein Molekulargewicht von 1716.13 aufweist.

Das Peptid wird anschließend in einem molaren Verhältnis 10:1 (Peptid zu Trägerprotein) mittels MH (RPLA) / SH-Peptid-Kopplung an Rinderplasmaalbumin als Trägerprotein gekoppelt.

Das Polyhapten wird abschließend in Anwesenheit von 40 mg/ml Trehalose gefriergetrocknet und zur Strichdosierung in demineralisiertem Wasser gelöst.

Das Polyhapten wird nachfolgend als PH, Tn I (27-43) [UZU-Cys-43]amid bezeichnet.

Das Goldkonjugatvlies 5 wird wie folgt hergestellt: Goldsol mit einem mittleren Partikeldurchmesser von ca. 40 nm wird nach der Methode von Frens (Frens, G., "Preparation of gold dispersions varying particle size: controlled nucleation for the regulation of the particle size in monodisperse gold suspensions" in Nature: Physical Science 241 (1973), 20 - 22) durch Reduktion einer 0,01 gewichtsprozentigen Tetrachlorgoldsäurelösung unter Kochen mittels Trinatriumcitrat hergestellt.

Die Antikörper-Goldkonjugat-Herstellung erfolgt in Anlehnung an die Methode von Roth, J. ("The colloidal gold marker system for light and electron microscopic cytochemistry" in Bullock, G.R. and Petrusz, P., Eds., "Techniques in Immunocytochemistry" Vol. 2, New York, Academic Press 1983, 216-284).

Dazu wird nach dem Abkühlen der Goldsollösung auf Raumtemperatur der pH-Wert des Goldsols mit 0,2 M K₂CO₃ so eingestellt, dass er um 0,5 pH-Einheiten über dem isoelektrischen Punkt des <cTn I>Antikörpers liegt. Der monoklonale <cTn I>Maus-Antikörper mit der Klonbezeichnung M155 kann kommerziell über die Katalog-Nr. 4T21 von der Fa. HyTest (Turku, Finnland) bezogen werden. Dieser monoklonale Antikörper erkennt das cTn I-Epitop aa 27-43.

Die optische Dichte (OD) des Goldsols (Extinktion bei 525 nm und 1 cm Lichtung) beträgt typischerweise 1,0. Der <cTn I>Antikörper wird in wässrig gelöster Form so zugegeben, dass dessen Konzentration nach Zugabe zur Goldsollösung bei 1,2 µg/ml liegt. Nach 30 Minuten Rühren bei Raumtemperatur wird das Goldkonjugat durch Zugabe einer hochkonzentrierten Rinderplasmaalbumin-Lösung (Endkonzentration in der Konjugatlösung 1 mg/ml) abgesättigt.

Nach weiterem 30-minütigem Rühren bei Raumtemperatur wird das nachgesättigte Goldkonjugat durch Ultrafiltration über eine 30 KDa-Membran gegen einen 20 mM Tris-Puffer pH 7,0 auf eine optische Dichte von typischerweise 20 aufkonzentriert. Die Konjugatlösung wird abschließend auf 100 µM Brij 35 und 0,05 Gew. % NaN₃ aufgestockt und bis zur Verwendung rollend bei 4° C gelagert.

Das Goldkonjugatvlies 5 wird mit nachfolgender Tränklösung imprägniert: 50 mM HEPES pH 7,5; 1,0 Gew. % Rinderplasmaalbumin; 1,0 % Saccharose; 0,1 % Tween 20; MAK<cTn I>M155-IgG - 40 nm Goldsol-Konjugat OD 4,5.

Dazu wird das Polyester-Zellwoll-Polyvinylalkohol-Mischvlies zunächst durch eine die Tränklösung enthaltende Wanne gezogen, anschließend durch zwei in einem Abstand von 250 µm angeordnete Edelstahlwalzen abgequetscht und abschließend mittels eines Umlufttrockners bei 60 °C getrocknet. Unter den beschriebenen Bedingungen beträgt die Tränkaufnahme des Vlieses typischerweise 240 ml/m².

Das Biotin-Konjugatvlies wird wie folgt hergestellt (6): Zur Herstellung des Biotin-Konjugates wird der monoklonale <cTn I>Maus-Antikörper mit der Klonbezeichnung 16A11 verwendet (kommerziell erhältlich über die Katalog Nr. 4T21 von der Fa. HyTest). Dieser monoklonale Antikörper erkennt das cTn 1-Epitop aa 87-91.

Zu einer Lösung von 10 mg/ml IgG in 0,1 M Kaliumphosphat pH 8,5 wird ein Succinimidesterderivat des Biotins in einem 8-fachen molaren Überschuss zugegeben. Der Ansatz wird für 90 Minuten bei 25° C unter Rühren inkubiert. Die Reaktion wird durch Aufstocken der Lösung mit Lysin auf eine Endkonzentration von 10 mM abgestoppt. Das überschüssige Biotinylierungsreagenz wird durch Dialyse entfernt und in Anwesenheit von 6 Gew. % Saccharose bis zur Verwendung bei -70° C gelagert.

Das Biotin-Konjugatvlies 6 wird mit nachfolgender Tränklösung imprägniert: 20 mM Tris pH 7,2; 50 mM NaCl; 1,0 Gew. % Rinderplasmaalbumin; 1,0 Gew. % Saccharose; 0,1 Gew. % Tween 20 und 7 mg/l MAK<cTn I>M-16A11-IgG-Biotin (XOSu 8:1). Tränkung und Trocknung erfolgte analog den bei der Goldkonjugatvlies-Herstellung verwendeten Bedingungen.

### b.) Bewertung der Teststreifen

Gemäß den unter a.) beschriebenen Verfahren wurden nachfolgende 3 Teststreifenvarianten gefertigt, die sich lediglich in der Zusammensetzung des Kontrollstrichs 8 unterscheiden. In den drei Varianten enthielten die Lösungen zur Herstellung der Kontrollstriche 8:
- Variante 1:: 1 mg/ml Polyhapten [PH, Tn I (27-43)]; Teststreifen mit klassischem Kontroll-strich.
- Variante 2:: 1 mg/ml Streptavidin; Teststreifen mit zweitem Nachweisstrich-Reagenz als Kontrollstrich.
- Variante 3:: 1 mg/ml Polyhapten und 1 mg/ml Streptavidin; Teststreifen mit erfindungs-gemäßem Kontrollstrich.

Wie bereits zuvor beschrieben, enthalten alle Teststreifenvarianten einen identischen Nachweisstrich 7 mit 4,5 mg/ml Streptavidin.

Zur Bewertung der Teststreifen wurde ein cTn I negatives Spenderblut verwendet, dem steigende Mengen an cTn I-C-T-Komplex (kommerziell erhältlich als Katalog-Nr. 8T62 von HyTest) zugesetzt wurden. Dazu wurde mittels Pipette 150 µl aufgestocktes Spenderblut pro Teststreifen als Probe auf das Biotinkonjugatvlies 6, das gleichzeitig als Probenauftragszone dient, aufgegeben und das Testergebnis nach 15 Minuten visuell auf Erscheinen eines Nachweisstriches und eines Kontrollstriches sowie zur Quantifizierung der Strichintensität mittels eines Messgerätes (Roche Cardiac Reader, Roche Diagnostics GmbH, Bestell-Nr. 1902229) reflektionsphotometrisch ausgewertet. Dabei steht 100 % Remission für das Ausbleiben eines Striches in der Nachweis- oder Kontrollstrich-Zone, wohingegen 20 % Remission für tief dunkelrote Striche steht, bei deren Intensität sich das Detektionssystem des Messgerätes in der Farbsättigung befindet.

Die Ergebnisse sind in nachfolgender Tabelle zusammengefasst.

| | **Signal (in [% Remission] bzw. visuell* positiv (+) oder negativ (-))** | | | |
|---|---|---|---|---|
| **cTn I-Konzentration [ng/ml]** | **Nachweisstrich** | **Kontrollstrich-Variante** | | |
| | | **1** | **2** | **3** |
| 0 | 100 (-) | 20 (+) | 100 (-) | 20 (+) |
| 1 | 65 (+) | 30 (+) | 80 (+) | 20 (+) |
| 10 | 25 (+) | 45 (+) | 60 (+) | 20 (+) |
| 50 | 18 (+) | 96 (+/-) | 40 (+) | 35 (+) |
| 250 | 30 (+) | 98 (-) | 65 (+) | 65 (+) |
| 1000 | 98 (-) | 100 (-) | 98 (-) | 98 (-) |

| | | | | |
|---|---|---|---|---|
| *Die Angabe (-) oder (+) bezieht sich auf die visuelle Ergebniszuordnung: (-) = kein sichtbarer Strich (+) = sichtbarer Strich | | | | |

Die Ergebnisse werden wie folgt interpretiert:

### Nachweisstrich:

Erwartungsgemäß nimmt mit steigender Analytkonzentration die Intensität des Nachweisstriches zunächst zu (Abnahme der Remissionswerte zwischen
1 ng/ml und 50 ng/ml cTn I), durchschreitet ein Intensitätsmaximum (18 % Remission, d. h. Farbsättigung), um dann bei extrem hohen Analytkonzentrationen aufgrund eines Sandwichformat bedingten High-Dose-Hook-Effektes wieder abzunehmen. Besagter High-Dose-Hook-Effekt führt am Nachweisstrich des Teststreifens bei 1000 ng/ml cTn I zu Remissionswerten nahe 100 % (visuell negativ) und damit zu einem falsch negativen Nachweisstrich.

### Variante 1: "klassischer Kontrollstrich":

In Abwesenheit von Analyt wird das Goldkonjugat via M155-Antikörper/Polyhapten (aa 27-43)-Bindung nahezu quantitativ am Kontrollstrich angereichert (20 % Remission). Mit steigender Analytkonzentration nimmt die Farbintensität des Kontrollstriches ab, da zum einen ein großer Teil des Goldkonjugates in Form des Sandwichkomplexes am Nachweisstrich abgefangen wird und da der am Nachweisstrich nicht abgefangene Goldkonjugatanteil durch die steigende Konzentration an anwesendem Analyt bereits abgesättigt ist und somit vom Polyhapten des Kontrollstriches nicht mehr ausreichend gebunden werden kann. In diesem Fall wandert das über den Nachweisstrich und über den Kontrollstrich chromatographierte Goldkonjugat in das flussabwärts liegende Waste-Vlies 2.

Ab 50 ng/ml cTn I in der Probe führt dies zu einem Versagen des Kontrollstriches (visuell negativ), obwohl der Nachweisstrich bei dieser Konzentration und auch bei 250 ng/ml noch eindeutig positiv ist.

Aufgrund des ausbleibenden Kontrollstriches muss der Anwender in diesem Fall jedoch fälschlicherweise vor einem ungültigen Testergebnis ausgehen.

Dieser Befund entspricht dem Stand der Technik und verdeutlicht somit die Limitationen eines klassischen Polyhapten-Kontrollstriches.

### Variante 2: "zweiter Nachweisstrich als Kontrollstrich":

Der als Kontrollstrich anzusehende zweite Streptavidin-Nachweisstrich versagt selbstverständlich in Abwesenheit von Analyt (in seiner Funktion als Kontrollstrich), da sich kein Sandwichkomplex ausgebildet hat. Das Testergebnis wäre somit als ungültig anzusehen, obwohl der negative erste Nachweisstrich die Abwesenheit des Analyts korrekt wiedergibt.

In Anwesenheit von Analyt detektiert der Streptavidin-Kontrollstrich analog zum Nachweisstrich den gebildeten Sandwichkomplex, wenn auch weniger intensiv, da der größere Teil des gebildeten Sandwichkomplexes flussaufwärts bereits vom Nachweisstrich gebunden wurde.

Bei sehr hohen Analytkonzentrationen (1000 ng/ml cTn I) versagt neben dem Nachweisstrich auch der Kontrollstrich, da beide dem gleichen Mechanismus unterliegen (vgl. oben zum High-Dose-Hook-Effekt).

### Variante 3: "erfindungsgemäßer Kontrollstrich":

Wie den Messwerten und visuellen Zuordnungen der Tabelle zu entnehmen ist, zeigt dieser Kontrollstrich in Abwesenheit von Analyt ein korrektes Testergebnis (via Polyhapten-Goldkonjugat-Mechanismus) an.

Im mittleren Konzentrationsbereich generiert der Kontrollstrich über beide immunologischen Mechanismen Signale, die sich aufaddieren.

Im oberen Konzentrationsbereich (50 - 250 ng/ml cTn I) generiert der Kontrollstrich das Messsignal im Wesentlichen über den Streptavidin-Biotin-Mechanismus (der "klassische" Polyhapten-Kontrollstrich befindet sich in diesem Fall bereits in der Analyt-Sättigung und versagt daher). Bei Vorliegen eines High-Dose-Hook-Effektes am Nachweisstrich (1000 ng/ml cTn I) versagt, wie erwünscht, auch der Kontrollstrich.

Aus der Summe dieser Eigenschaften wird ersichtlich, dass dieser neuartige Kontrollstrich-Mechanismus den bisher bekannten technischen Ausführungen überlegen ist.

## Patentansprüche

1. Testelement zur Durchführung eines immunologischen Sandwichtestes zur Bestimmung eines Analyten aus einer flüssigen Probe enthaltend
- eine Reagenzzone, die ein durch die flüssige Probe lösliches Konjugat aus einem Analyt-Bindepartner und einem Label, das direkt oder indirekt visuell, optisch oder elektrochemisch nachweisbar ist, enthält;
- eine Nachweiszone, die einen permanent immobilisierten Bindepartner für den Analyten oder für den Analyten enthaltende Komplexe enthält; und
- eine Kontrollzone, die einen permanent immobilisierten Bindepartner für das Konjugat aus Analyt-Bindepartner und Label enthält,
**dadurch gekennzeichnet, dass**
die Kontrollzone weiterhin einen oder mehrere permanent immobilisierte(n) Bindepartner für den Analyten oder für den Analyten enthaltende Komplexe enthält.

2. Testelement gemäß Anspruch 1, **dadurch gekennzeichnet, dass**
die permanent immobilisierten Bindepartner für den Analyten oder für den Analyten enthaltende Komplexe in der Nachweiszone und in der Kontrollzone gleich sind.

3. Testelement gemäß Anspruch 1, **dadurch gekennzeichnet, dass**
die permanent immobilisierten Bindepartner für den Analyten oder für den Analyten enthaltende Komplexe in der Nachweiszone und in der Kontrollzone ungleich sind.

4. Testelement gemäß einem der vorherigen Ansprüche, **dadurch gekennzeichnet, dass**
der permanent immobilisierte Bindepartner für das Konjugat aus Analyt-Bindepartner und Label in der Kontrollzone ein Polyhapten ist.

5. Testelement gemäß einem der vorherigen Ansprüche, **dadurch gekennzeichnet, dass**
der permanent immobilisierte Bindepartner für den Analyten oder für den Analyten enthaltende Komplexe in der Nachweiszone ausgewählt ist aus der Gruppe umfassend
- Antikörper oder immunologisch aktives Fragment eines Antikörpers gegen den Analyten;
- Antigene, Haptene oder Polyhaptene; und
- Streptavidin oder Polystreptavidin.

6. Testelement gemäß einem der vorherigen Ansprüche, **dadurch gekennzeichnet, dass**
der permanent immobilisierte Bindepartner für den Analyten oder für den Analyten enthaltende Komplexe in der Kontrollzone ausgewählt ist aus der Gruppe umfassend
- Antikörper oder immunologisch aktives Fragment eines Antikörpers gegen den Analyten;
- Antigene, Haptene oder Polyhaptene; und
- Streptavidin oder Polystreptavidin.

7. Testelement gemäß einem der vorherigen Ansprüche, **dadurch gekennzeichnet, dass**
der permanent immobilisierte Bindepartner für den Analyten in der Nachweiszone ein Antikörper oder immunologisch aktives Fragment eines Antikörpers gegen den Analyten ist; und
der permanent immobilisierte Bindepartner für den Analyten in der Kontrollzone ein Antikörper oder immunologisch aktives Fragment eines Antikörpers gegen den Analyten ist.

8. Testelement gemäß einem der vorherigen Ansprüche, **dadurch gekennzeichnet, dass**
der permanent immobilisierte Bindepartner für den Analyten enthaltende Komplexe in der Nachweiszone Streptavidin oder Polystreptavidin ist; und
der permanent immobilisierte Bindepartner für den Analyten enthaltende Komplexe in der Kontrollzone Streptavidin oder Polystreptavidin ist.

9. Testelement gemäß Anspruch 1, **dadurch gekennzeichnet, dass**
der permanent immobilisierte Bindepartner für den Analyten enthaltende Komplexe in der Nachweiszone Streptavidin oder Polystreptavidin ist; und
der permanent immobilisierte Bindepartner für den Analyten und/oder für den Analyten enthaltende Komplexe in der Kontrollzone
- ein Antikörper oder immunologisch aktives Fragment eines Antikörpers gegen den Analyten; und/oder
- Streptavidin oder Polystreptavidin ist.

10. Verfahren zum Nachweis eines Analyten aus einer flüssigen Probe, wobei
- die Probe auf ein Testelement gemäß einem der Ansprüche 1 bis 9 aufgegeben wird;
- der Analyt - sofern in der Probe vorhanden - mit dem gelabelten Konjugat zu einem Analyt-Konjugat-Komplex reagiert;
- der Analyt-Konjugat-Komplex in der Nachweiszone an den permanent immobilisierten Bindepartner für den Analyten oder für den Analyten enthaltende Komplexe bindet; und
- gelabeltes Konjugat, das nicht mit dem Analyten reagiert hat, in der Kontrollzone an den permanent immobilisierten Bindepartner für das Konjugat bindet; und
- Analyt-Konjugat-Komplex in der Kontrollzone an den permanent immobilisierten Bindepartner für den Analyten oder für den Analyten enthaltende Komplexe bindet

## Claims

1. Test element for carrying out an immunological sandwich test for determining an analyte from a liquid sample containing
- a reagent zone which contains a conjugate of an analyte binding partner and a label which can be detected directly or indirectly by visual, optical or electrochemical means wherein said conjugate can be dissolved by the liquid sample;
- a detection zone which contains a permanently immobilized binding partner for the analyte or for complexes containing the analyte; and
- a control zone which contains a permanently immobilized binding partner for the conjugate of analyte binding partner and label
**characterized in that**
the control zone additionally contains one or more permanently immobilized binding partner(s) for the analyte or for complexes containing the analyte.

2. Test element according to claim 1, **characterized in that**
the permanently immobilized binding partners for the analyte or for complexes containing the analyte are the same in the detection zone and in the control zone.

3. Test element according to claim 1, **characterized in that**
the permanently immobilized binding partners for the analyte or for complexes containing the analyte are not the same in the detection zone and in the control zone.

4. Test element according to one of the previous claims, **characterized in that** the permanently immobilized binding partner for the conjugate of analyte binding partner and label in the control zone is a polyhapten.

5. Test element according to one of the previous claims, **characterized in that** the permanently immobilized binding partner for the analyte or for complexes containing the analyte in the detection zone is selected from the group comprising
- antibody or immunologically active fragment of an antibody to the analyte,
- antigens, haptens or polyhaptens, and
- streptavidin or polystreptavidin.

6. Test element according to one of the previous claims, **characterized in that** the permanently immobilized binding partner for the analyte or for complexes containing the analyte in the control zone is selected from the group comprising
- antibody or immunologically active fragment of an antibody to the analyte,
- antigens, haptens or polyhaptens, and
- streptavidin or polystreptavidin.

7. Test element according to one of the previous claims, **characterized in that** the permanently immobilized binding partner for the analyte in the detection zone is an antibody or an immunologically active fragment of an antibody to the analyte and
the permanently immobilized binding partner for the analyte in the control zone is an antibody or immunologically active fragment of an antibody to the analyte.

8. Test element according to one of the previous claims, **characterized in that** the permanently immobilized binding partner for complexes containing the analyte in the detection zone is streptavidin or polystreptavidin and
the permanently immobilized binding partner for complexes containing the analyte in the control zone is streptavidin or polystreptavidin.

9. Test element according to claim 1, **characterized in that**
the permanently immobilized binding partner for complexes containing the analyte in the detection zone is streptavidin or polystreptavidin and
the permanently immobilized binding partner for the analyte and/or for complexes containing the analyte in the control zone is
- an antibody or immunologically active fragment of an antibody to the analyte, and/or
- streptavidin or polystreptavidin.

10. Method for detecting an analyte from a liquid sample, wherein
- the sample is applied to a test element according to one of the claims 1 to 9,
- the analyte - if present in the sample - reacts with the labelled conjugate to form an analyte-conjugate complex,
- the analyte-conjugate complex binds in the detection zone to the permanently immobilized binding partner for the analyte or for complexes containing the analyte, and
- labelled conjugate that has not reacted with the analyte binds in the control zone to the permanently immobilized binding partner for the conjugate, and
- analyte-conjugate complex in the control zone binds to the permanently immobilized binding partner for the analyte or for complexes containing the analyte.

## Revendications

1. Elément de test pour réaliser un test immunologique en sandwich en vue de déterminer un analyte d'un échantillon liquide contenant
- une zone de réactif, qui contient un conjugué soluble à l'aide de l'échantillon liquide, constitué d'un partenaire de liaison d'analyte et d'un marqueur, qui peut être détecté directement ou indirectement de façon visuelle, optique ou électrochimique;
- une zone de détection qui contient un partenaire de liaison immobilisé en permanence pour l'analyte ou pour le complexe contenant l'analyte; et
- une zone de contrôle, qui contient un partenaire de liaison immobilisé en permanence pour le conjugué constitué d'un partenaire de liaison d'analyte et d'un marqueur,
**caractérisé en ce que**
la zone de contrôle contient en outre un ou plusieurs partenaires de liaison immobilisés en permanence pour l'analyte ou pour le complexe contenant l'analyte.

2. Elément de test selon la revendication 1, **caractérisé en ce que** les partenaires de liaison immobilisés en permanence pour l'analyte ou pour le complexe contenant l'analyte sont les mêmes dans la zone de détection et dans la zone de contrôle.

3. Elément de test selon la revendication 1, **caractérisé en ce que** les partenaires de liaison immobilisés en permanence pour l'analyte ou pour le complexe contenant l'analyte sont différents dans la zone de détection et dans la zone de contrôle.

4. Elément de test selon l'une quelconque des revendications précédentes, **caractérisé en ce que** le partenaire de liaison immobilisé en permanence pour le conjugué constitué d'un partenaire de liaison d'analyte et d'un marqueur dans la zone de contrôle, est un polyhaptène.

5. Elément de test selon l'une quelconque des revendications précédentes, **caractérisé en ce que** le partenaire de liaison immobilisé en permanence pour l'analyte ou pour le complexe contenant l'analyte dans la zone de détection est choisi dans le groupe comprenant
- les anticorps ou un fragment immunologiquement actif d'un anticorps contre l'analyte;
- les antigènes, les haptènes ou les polyhaptènes ; et
- la streptavidine ou la polystreptavidine.

6. Elément de test selon l'une quelconque des revendications précédentes, **caractérisé en ce que** le partenaire de liaison immobilisé en permanence pour l'analyte ou pour le complexe contenant l'analyte dans la zone de contrôle est choisi dans le groupe contenant
- les anticorps ou un fragment immunologiquement actif d'un anticorps contre l'analyte ;
- les antigènes, les haptènes ou les polyhaptènes ; et
- la streptavidine ou la polystreptavidine.

7. Elément de test selon l'une quelconque des revendications précédentes, **caractérisé en ce que** le partenaire de liaison immobilisé en permanence pour l'analyte dans la zone de détection est un anticorps ou un fragment immunologiquement actif d'un anticorps contre l'analyte ; et
le partenaire immobilisé en permanence pour l'analyte dans la zone de contrôle est un anticorps ou un fragment immunologiquement actif d'un anticorps contre l'analyte.

8. Elément de test selon l'une quelconque des revendications précédentes, **caractérisé en ce que** le partenaire de liaison immobilisé en permanence pour le complexe contenant l'analyte dans la zone de détection est la streptavidine ou la polystreptavidine ; et
le partenaire de liaison immobilisé en permanence pour le complexe contenant l'analyte dans la zone de contrôle est la streptavidine ou la polystreptavidine.

9. Elément de test selon la revendication 1, **caractérisé en ce que** le partenaire de liaison immobilisé en permanence pour le complexe contenant l'analyte dans la zone de détection est la streptavidine ou la polystreptavidine ; et
le partenaire de liaison immobilisé en permanence pour l'analyte et/ou pour le complexe contenant l'analyte dans la zone de contrôle est
- un anticorps ou un fragment immunologiquement actif d'un anticorps contre l'analyte ; et/ou
- la streptavidine ou la polystreptavidine.

10. Procédé pour détecter un analyte provenant d'un échantillon liquide, dans lequel
- l'échantillon est posé sur un élément de test selon l'une quelconque des revendications 1 à 9 ;
- l'analyte dans la mesure où il est présent dans l'échantillon, réagit avec le conjugué marqué en donnant un complexe analyte/conjugué ;
- le complexe analyte/conjugué dans la zone de détection se lie au partenaire de liaison immobilisé en permanence pour l'analyte ou pour le complexe contenant l'analyte ; et
- un conjugué marqué qui n'a pas réagi avec l'analyte, dans la zone de contrôle se lie au partenaire de liaison immobilisé en permanence pour le conjugué ; et
- un complexe analyte/conjugué dans la zone de contrôle se lie au partenaire de liaison immobilisé en permanence pour l'analyte ou pour le complexe contenant l'analyte.
